# EUROPEAN PATENT SPECIFICATION

(11) **EP 4 094 259 B1**
(45) Date of publication and mention of the grant of the patent: **24.12.2025**
(21) Application number: 21700612.1
(22) Date of filing: 22.01.2021
(51) Int. Cl.: G16B 20/00, G16B 40/20

(54) **A METHOD OF ESTABLISHING AN EPIGENETIC CLOCK FOR AVIAN SPECIES**
VERFAHREN ZUR FESTLEGUNG EINER EPIGENETISCHEN UHR FÜR VOGELARTEN
PROCÉDÉ D'ÉTABLISSEMENT D'UNE HORLOGE ÉPIGÉNÉTIQUE POUR UNE ESPÈCE AVIAIRE

(30) Priority: 24.01.2020 EP 20153518
(43) Date of publication of application: 30.11.2022
(73) Proprietor: Evonik Operations GmbH, 45128 Essen (DE)
(72) Inventor: RADDATZ, Günter, 69126 Heidelberg (DE); LYKO, Frank, 69493 Hirschberg an der Bergstraße (DE); BÖHL, Florian, 60151 Neckargemünd (DE); KAPPEL, Andreas, 61479 Glashütten (DE); IGWE, Emeka Ignatius, 80333 München (DE); THIEMANN, Frank, 48031 Nottuln (DE); PELZER, Stefan, 33335 Gütersloh (DE)
(74) Representative: Evonik Patent Association
(86) International application number: PCT/EP2021/051434
(87) International publication number: WO 2021/148593

(56) References cited:
- WO-A1-2018/146482
- US-A1- 2020 010 908
- THOMAS M. STUBBS ET AL: "Multi-tissue DNA methylation age predictor in mouse", GENOME BIOLOGY, vol. 18, no. 1, 11 April 2017 (2017-04-11), XP055457746, DOI: 10.1186/s13059-017-1203-5
- SIPENG SHEN ET AL: "Seven-CpG-based prognostic signature coupled with gene expression predicts survival of oral squamous cell carcinoma", CLINICAL EPIGENETICS, BIOMED CENTRAL LTD, GB, vol. 9, no. 1, 24 August 2017 (2017-08-24), pages 1 - 11, XP021248267, ISSN: 1868-7075, DOI: 10.1186/S13148-017-0392-9
- STEVE HORVATH ET AL: "Erratum to: DNA methylation age of human tissues and cell types", GENOME BIOLOGY, 13 May 2015 (2015-05-13), England, pages 96 - 96, XP055750470, Retrieved from the Internet <URL:https://genomebiology.biomedcentral.com/track/pdf/10.1186/gb-2013-14-10-r115.pdf> DOI: 10.1186/s13059-015-0649-6
- MORRIS TIFFANY J. ET AL: "Analysis pipelines and packages for Infinium HumanMethylation450 BeadChip (450k) data", METHODS, vol. 72, 15 January 2015 (2015-01-15), NL, pages 3 - 8, XP055790540, ISSN: 1046-2023, DOI: 10.1016/j.ymeth.2014.08.011

## Description

### Field of the Invention

The present invention describes methods for establishing whole-tissue epigenetic clocks for avian species. The thus-obtained epigenetic clocks are particularly robust, generalizable and provide high specificity, accuracy and precision.

### Background of the Invention

Avian species, and in particular *Galliformes,* such as chicken (*Gallus gallus*) are a significant source of commercially produced meat and eggs. Factors that influence the growth, pathogen resistance and meat quality of chicken are thus of considerable scientific and economical interest. Extensive genome-wide association studies have been conducted to elucidate the underlying genetic framework. Epigenetic modifications provide an important complement and extension to genetic variants but have remained relatively underexplored in chicken.

Animal methylomes can be highly diverse, ranging from certain insect genomes with sparse methylation patterns and only tens of thousands of methylation marks to mammalian genomes with dense methylation patterns and tens of millions of methylation marks. Until now, only little is known about the genome-wide DNA methylation patterns of non-mammalian vertebrates, and particularly of birds.

DNA methylation correlates with ageing processes and represents an epigenetic modification with a high specificity for CpG dinucleotides (5'-C-phosphate-G-3'), i.e. regions of DNA where a cytosine nucleotide is followed by a guanine nucleotide in the linear sequence of bases along its 5'→ 3 direction. The set of genomic methylation modifications constitutes the methylome of a given cell.

Low-methylated regions (LMRs) represent a key feature of the dynamic methylome. LMRs are local reductions in the DNA methylation landscape and represent CpG-poor distal regulatory regions that often reflect the binding of transcription factors and other DNA-binding proteins. LMRs were originally described in the mouse (Stadler et al. Nature 480, 490-495 (2011)). Evolutionary conservation of LMRs beyond mammals has remained unexplored.

Age-correlated DNA methylation changes at discrete sets of CpGs in the human genome have been identified and used to predict age (Horvath, S. (2013). DNA methylation age of human tissues and cell types. Genome Biology 14:3156). These "epigenetic clocks" can estimate the DNA methylation age in specific tissues or tissue-independently and can predict mortality and time to death.

Epigenetic age is highly correlated with chronological age but can also respond to environmental factors that accelerate or decelerate ageing processes, resulting in substantial deviations from chronological age.

Epigenetic age acceleration (epigenetic age > chronological age) suggests that the underlying tissue ages faster than expected on the basis of chronological age, whereas a negative value (epigenetic age < chronological age, age deceleration) suggests that the tissue ages slower than would be expected. Epigenetic age acceleration is associated with a great number of age-related conditions and diseases, such as inflammatory processes.

In view of those conditions accelerating the biological/epigenetic age, age-correlated performance biomarkers are particularly useful tools for animal farming, as they facilitate monitoring large groups of animals and provide objective quality assurance. Avian species present a unique challenge for performance biomarker development, as they combine considerable economic importance with a relatively short lifespan.

WO 2018/146482 A1 discloses a method for calculating the age of a biological sample obtained from a subject, in particular a mouse. It also discloses ageing biomarkers, a kit comprising biosensors capable of detecting said biomarkers, a method of assessing the effect of a biological intervention upon the age of a subject and a method of screening for an anti-ageing agent.

Accordingly, it was the objective of the present invention to provide a method of establishing a whole-tissue epigenetic clock for avian species that may be used as a performance biomarker for the respective avian species and that provides robustness and generalizability, and at the same time high specificity, accuracy and precision.

### Summary of the Invention

The present invention provides a computer-implemented method of establishing an epigenetic clock for an avian species, according to claim 1.

Further, it provides a computer program loaded into a memory of a computer, implementing the aforementioned method.

Finally, the present invention pertains to a tangible, computer-readable medium according to claim 13.

### Detailed Description of the Invention

The inventors have identified three previously unknown confounding factors for methylation clocks for avian species:
- Genetic polymorphisms are known to strongly affect epigenetic association studies. For example a genetic polymorphism in a CpG site results in a sequence that cannot be methylated anymore and will therefore be scored as unmethylated. However, the underlying effect does not represent an age-related change, but a confounding factor.
- Sex chromosomes are known to carry sex-specific methylation marks that facilitate dosage compensation of heterogametic sex chromosomes. This effect can confound the identification of age-related methylation changes.
- Different tissues show different maturation stages at the time of birth and age at different speeds. As such, the normalization of aging trajectories can substantially improve the performance and robustness of a multi-tissue clock.

The inventors have found that robustness, specificity, accuracy and precision of epigenetic clocks for avian species can be significantly improved by (i) excluding from the initially identified clock CpG sites all CpG sites associated with single nucleotide polymorphisms (SNPs), (ii.) excluding all CpG sites located on the sex chromosomes (Z and W), and (iii.) normalizing the CpG methylation values. Accordingly, the present invention provides a computer-implemented method of establishing an epigenetic clock for an avian species, according to claim 1.

The term "CpG site", "clock CpG" or "CpG location" as used in the context of the present invention refers to a CpG position that is potentially methylated. Methylation typically occurs in a CpG containing nucleic acid. The CpG containing nucleic acid may be present in, e.g. a CpG island, a CpG doublet, a promoter, an intron, or an exon of a gene or in an intergenic region. For instance, the potential methylation sites may encompass the promoter/enhancer regions of the indicated genes.

According to method step (a), CpG sites within the genomic DNA of the avian species were identified and the methylation level of those CpG sites is determined.

Accordingly, method step (a) involves a DNA methylation profiling process, preferably bisulfite sequencing. Therein, cytosine residues in the genomic DNA are transformed to uracil, while 5-methylcytosine residues in the genomic DNA are not transformed to uracil.

Whole genome bisulfite sequencing is a genome-wide analysis of DNA methylation based on the sodium bisulfite conversion of genomic DNA, which is then sequenced on a next-generation sequencing platform. The sequences are then re-aligned to the reference genome to determine methylation states of the CpG dinucleotides based on mismatches resulting from the conversion of unmethylated cytosines into uracil.

For example, methylation levels can be measured using the commercial Illumina^{™} platform.

To quantify the methylation level, various established protocols may be used to calculate the beta value of methylation, which equals the fraction of methylated cytosines in a specific location.

The specific CpG sited within the genomic DNA of the avian species are located within LMRs ("LMR clock"). Details for establishing the genome-wide clock and the LMR clock, resp., are provided below.

The genomic DNA is obtained from a plurality of different sample materials deriving from the avian species and representing specific time points within the chronological lifespan of this avian species. As an example, the sample material may be stratified into four tissue (breast, ileum, spleen and jejunum) and three age (3 d, 15 d, 34 d) groups. Details regarding suitable sample materials will be provided below. Ideally, the sample material covers the entire life cycle of the avian species under investigation.

In method step (b), all CpG sites associated with single nucleotide polymorphisms (SNPs) were excluded from the CpG sites identified in step (a.). SNPs can be determined using standard procedures known in the art, such as whole-genome sequencing. Alternatively, SNPs in the genome of selected species are publicly available in databases, such as dbSNP (https://www.ncbi.nlm.nih.gov/snp/).

In method step (c), all CpG sites located on the sex chromosomes (Z and W) were excluded from the CpG sites obtained in step (b.). Birds have female heterogamy with Z and W sex chromosomes (hhttps://www.ncbi.nlm.nih.gov/pmc/articles/PMC2567362/). The chromosome names are usually annotated in the assembly of a species. As an example for chicken (*Gallus gallus*), the chromosomal location of a CpG can be derived from the annotation of the *Gallus gallus* genome assembly version 5.0 (https://www.ebi.ac.uk/ena/data/view/GCA_000002315.3).

In method step (d), a tissue-specific normalization step for the CpG sites obtained in step (c.) is performed. Normalization is performed by computing for every CpG the average methylation value over all samples from the same tissue and subtracting the thus-obtained value from the value of this CpG (for the LMR clock: by computing for every LMR the average methylation value over all samples from the same tissue and subtracting the thus-obtained value from the value of this LMR). This normalization is necessitated by the different aging trajectories of individual tissues.

The CpG sites obtained in step (d), i.e. the CpG sites remaining after correction of the above-mentioned confounding factors, were finally correlated with chronological age using a penalized regression model (method step (e)).

The plurality of different biological sample materials deriving from the avian species and representing specific time points within the chronological lifespan of this avian species may include material selected from the group consisting of body fluids, excremental material, tissue material and feather material. In one embodiment of the invention, the plurality of different biological sample materials deriving from the avian species and representing specific time points within the chronological lifespan of this avian species includes only one specific tissue, or maximally four different tissues.

Preferably, the plurality of different biological sample materials deriving from the avian species and representing specific time points within the chronological lifespan of this avian species includes at least four different tissues and preferably exactly four different tissues.

In one embodiment of the present invention, the plurality of different biological sample materials deriving from the avian species and representing specific time points within the chronological lifespan of this avian species comprises or consists of tissue material selected from muscle tissue; organ tissue, such as gut tissue; and skin tissue.

Preferably, the plurality of different biological sample materials deriving from the avian species and representing specific time points within the chronological lifespan of this avian species includes or consists of breast tissue, spleen tissue, ileum tissue and jejunum tissue. The aforementioned set of tissues is particularly preferred as it represents a biologically diverse and commercially relevant set of tissues.

The plurality of different biological sample materials deriving from the avian species and representing specific time points within the chronological lifespan of this avian species is preferably selected to represent ages ranging between day 3 and day 63, in particular between day 4 and day 42 and preferably between day 5 and day 35.

For example, the life cycle of chicken starts with eggs taken from parent birds in the hatchery which are then incubated at a constant temperature for 21 days until the birds hatch, though at this stage the precocial chicken might be up to 72 hours old they are called one-day chicken. These chickens are separated by sexes and the female birds are kept for approx. one year for laying eggs. The lifespan for broiler chicken is significantly shorter and varies between 21 days and up to 170 days. An average US broiler is slaughtered after 47 days at a slaughter weight of 2.6 kg while in Europe the average slaughter age is at 42 days (at a weight of 2.5 kg).

### Establishment of a genome-wide Clock ("CpG Clock")

As indicated above, the specific CpG sites within the genomic DNA of the avian species may be distributed over the whole genome of the avian species ("genome-wide clock"). In this case, the CpGs were preferably restricted to a strand-specific coverage of at least 10.

### Establishment of an LMR Clock

The specific CpG sites within the genomic DNA of the avian species are distributed within low methylated regions (LMRs) in the genome of the avian species. In this case, method step (a) includes a step of computing LMRs individually for the different tissues.

Suitable LMR computing programs are known in the art, for example MethylSeekR (Burger L, Gaidatzis D, Schubeler D, Stadler MB. Identification of active regulatory regions from DNA methylation data. Nucleic Acids Res 41, e155 (2013)).

For establishing the LMR clock, the specific CpG sites within the genomic DNA of the avian species were preferably restricted to a strand-specific coverage of at least greater than 5.

An LMR clock allows the conceptual interpretation of the selected features, as LMRs represent transcription factor binding sites. This represents an important advantage compared to all-CpG clocks. Furthermore, LMR clocks are more robust to noise, as the features represent averages over regions and noise cancels out.

In addition to the above, the present invention pertains to a computer program loaded into a memory of a computer, implementing any one of the above-described method.

Finally, the present invention relates to a tangible, computer-readable medium according to claim 13.

Applications of the methods according to the invention are for example development of new epigenetic clocks as biomarkers (i) aiding in evaluation of the health status of avian species (individual or population) (ii) monitoring the progress or reoccurrence of clinical and sub-clinical disorders or (iii) studying the effects of medication, feed compounds and/or special diets on the biological age - and thus on the health status of the respective avian species.

### Examples

### METHODS

### Samples

Animals were stratified into four tissue (breast, ileum, spleen and jejunum) and three age (3 d, 15 d, 34 d) groups, in case of jejunum 14 d, 16 d and 35 d. From each of these 12 groups, DNA was prepared from three independent animals, resulting in 36 genomic DNA samples.

### Whole-genome bisulfite sequencing

Whole-genome bisulfite sequencing libraries were prepared using the Accel-NGS Methyl-Seq DNA Library Kit from Swift Biosciences. Two sequencing libraries were barcoded onto one sequencing lane. Sequencing was performed on an Illumina HiSeq X platform using a standard paired-end sequencing protocol with 105 nucleotides read length.

### Read mapping

Reads were trimmed and mapped with BSMAP 2.5 (Xi Y, Li W. 2009. BSMAP: whole genome bisulfite sequence MAPping program. BMC Bioinformatics 10:232. doi:10.1186/1471-2105-10-232.) using the *Gallus gallus* genome assembly version 5.0 (https://www.ebi.ac.uk/ena/data/view/GCA_000002315.3) as a reference sequence. Duplicates were removed using the Picard tool (http://broadinstitute.github.io/picard). Methylation ratios were determined using a Python script (methratio.py) distributed together with the BSMAP package by dividing the number of reads having a methylated CpG at a certain genomic position by the number of all reads covering this position.

### Normalization and SNP filtering of the methylation data

All CpGs which are listed as SNPs in the database dbSNP (https://www.ncbi.nlm.nih.gov/snp/) for the *Gallus gallus* genome were filtered out. All CpGs and LMRs mapping to the Galliformes sex Chromosomes W and Z were filtered out and removed from the data sets. For the genome-wide clock, the analysis was restricted to CpGs that showed a strand specific coverage of greater than 10 in every of the sequenced samples, resulting in a set of 257,913 CpGs. Then the data were normalized by computing for every CpG the average methylation value over all samples from the same tissue and subtracted this value from the methylation value of this CpG. For the LMR clock, the analysis was restricted to CpGs within low-methylated regions that showed a strand specific coverage of greater than 5 in every of the sequenced samples, resulting in a set of 67,651 LMRs. The average methylation values of these LMRs were computed and normalized by computing for every LMR the average value over all samples from the same tissue and subtracting this value from the value of this LMR.

### Establishment of a chicken DNA methylation clock

Then a penalized regression model (implemented in the R package glmnet [https://cran.r-project.org/web/packages/glmnet/]) was applied to regress the chronological age of the animals on the normalized methylation values of the CpG probes. In the case of the LMR clock a penalized regression model was applied to regress the chronological age of the animals on the normalized average methylation values of the LMRs.

### RESULTS

### Genome-wide clock

The alpha parameter of glmnet was varied in a range between 0 and 1 and chosen as 0.7 (elastic net regression), because this value led to a fit that was close to the best fit and a manageable amount of CpGs. The lambda value was chosen using cross-validation on the training data as 0.4016. This identified a set of 45 CpGs together with corresponding beta values, which define the weights for these CpGs used in the chicken methylation clock. The mean squared error of 6-fold crossvalidation using the values of 0.7 for alpha and 0.4016 for lambda was 11.538. This indicates that a new sample can be predicted with an error of about 3.4 days. In order to apply the clock to a new sample the methylation ratios of this sample at the 45 clock CpGs have to be provided and the command predict.cv of the package glmnet with the trained clock has to be performed.

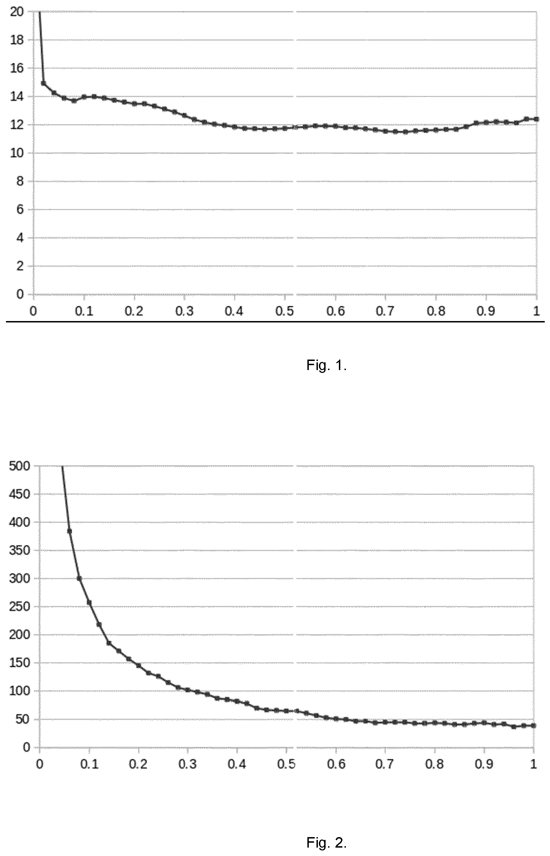

Fig. 1 shows the mean squared error of a trained clock for given alpha at value of lambda leading to the minimal error.

Fig. 2 shows the number of CpGs for given alpha at value of lambda leading to the minimal error.

**Table 1: Clock CpGs (genome-wide methylation, alpha = 0.7, lambda = 0.4016, #CpG's: 45).**

| ¹: Correction factors of the different tissues. The respective value has to be subtracted. | | | | | | | |
|---|---|---|---|---|---|---|---|
| ID | chrom | position | weight | Ileum ¹ | Spleen ¹ | Breast ¹ | Jejunum ¹ |
| 1 | chr1 | 26806096 | -0.333 | 0.636 | 0.475 | 0.464 | 0.64 |
| 2 | chr1 | 27051068 | -1.207 | 0.363 | 0.124 | 0.445 | 0.235 |
| 3 | chr1 | 79412910 | -3.879 | 0.467 | 0.438 | 0.573 | 0.414 |
| 4 | chr1 | 193007724 | -0.894 | 0.504 | 0.181 | 0.398 | 0.44 |
| 5 | chr2 | 84879641 | 2.595 | 0.381 | 0.665 | 0.191 | 0.415 |
| 6 | chr2 | 139780944 | -0.004 | 0.32 | 0.198 | 0.053 | 0.182 |
| 7 | chr3 | 9654592 | -2.179 | 0.503 | 0.328 | 0.698 | 0.589 |
| 8 | chr3 | 23119819 | -2.285 | 0.282 | 0.251 | 0.31 | 0.292 |
| 9 | chr3 | 32240754 | 2.209 | 0.256 | 0.244 | 0.148 | 0.264 |
| 10 | chr3 | 55893779 | -3.285 | 0.528 | 0.563 | 0.673 | 0.564 |
| 11 | chr3 | 55933564 | -0.301 | 0.335 | 0.302 | 0.649 | 0.165 |
| 12 | chr4 | 20608622 | -0.825 | 0.547 | 0.512 | 0.554 | 0.728 |
| 13 | chr4 | 48345505 | 0.468 | 0.285 | 0.435 | 0.239 | 0.304 |
| 14 | chr4 | 70292571 | -0.001 | 0.254 | 0.235 | 0.561 | 0.332 |
| 15 | chr5 | 1942965 | 3.015 | 0.268 | 0.532 | 0.178 | 0.322 |
| 16 | chr5 | 1942982 | 2.248 | 0.334 | 0.562 | 0.174 | 0.397 |
| 17 | chr5 | 12844701 | -0.238 | 0.583 | 0.435 | 0.711 | 0.691 |
| 18 | chr5 | 16850281 | 1.412 | 0.651 | 0.784 | 0.654 | 0.723 |
| 19 | chr5 | 17507391 | -3.468 | 0.261 | 0.197 | 0.115 | 0.351 |
| 20 | chr5 | 39037892 | 1.739 | 0.476 | 0.506 | 0.379 | 0.61 |
| 21 | chr5 | 54227250 | -1.625 | 0.225 | 0.358 | 0.361 | 0.28 |
| 22 | chr5 | 58662889 | 5.718 | 0.46 | 0.621 | 0.364 | 0.503 |
| 23 | chr6 | 5240214 | -0.287 | 0.262 | 0.317 | 0.196 | 0.213 |
| 24 | chr6 | 7819244 | 4.26 | 0.209 | 0.511 | 0.234 | 0.188 |
| 25 | chr6 | 12024016 | -2.447 | 0.662 | 0.24 | 0.575 | 0.515 |
| 26 | chr6 | 12065954 | 1.12 | 0.286 | 0.388 | 0.249 | 0.325 |
| 27 | chr7 | 9815074 | -5.1 | 0.726 | 0.46 | 0.738 | 0.655 |
| 28 | chr7 | 11137846 | -0.002 | 0.367 | 0.286 | 0.587 | 0.326 |
| 29 | chr7 | 14040077 | -1.945 | 0.431 | 0.309 | 0.357 | 0.366 |
| 30 | chr7 | 21995171 | -2.653 | 0.192 | 0.057 | 0.244 | 0.137 |
| 31 | chr7 | 30586853 | 0.837 | 0.335 | 0.391 | 0.176 | 0.501 |
| 32 | chr8 | 3444574 | 1.024 | 0.255 | 0.654 | 0.388 | 0.256 |
| 33 | chr8 | 8196471 | 0.618 | 0.56 | 0.802 | 0.691 | 0.565 |
| 34 | chr8 | 18912606 | -1.112 | 0.442 | 0.333 | 0.599 | 0.542 |
| 35 | chr8 | 27250408 | -0.755 | 0.473 | 0.413 | 0.394 | 0.735 |
| 36 | chr10 | 20035839 | -0.002 | 0.251 | 0.14 | 0.142 | 0.234 |
| 37 | chr11 | 7627454 | 0.396 | 0.593 | 0.601 | 0.222 | 0.672 |
| 38 | chr14 | 9143159 | -3.085 | 0.519 | 0.34 | 0.564 | 0.355 |
| 39 | chr14 | 9143204 | -2.843 | 0.678 | 0.401 | 0.615 | 0.388 |
| 40 | chr15 | 201524 | 6.892 | 0.596 | 0.634 | 0.3 | 0.559 |
| 41 | chr15 | 8945553 | -13.223 | 0.766 | 0.724 | 0.87 | 0.542 |
| 42 | chr17 | 1673086 | -0.441 | 0.616 | 0.305 | 0.472 | 0.669 |
| 43 | chr19 | 7327224 | 5.149 | 0.657 | 0.492 | 0.266 | 0.648 |
| 44 | chr23 | 172291 | -0.279 | 0.646 | 0.538 | 0.562 | 0.479 |
| 45 | chr23 | 5568087 | -1.692 | 0.277 | 0.183 | 0.18 | 0.255 |
| Intercept of linear model equation found by glmnet: 17.365 | | | | | | | |

### LMR clock

### Example 1:

The alpha parameter of glmnet was varied in a range between 0 and 1 and chosen as 0.84 (elastic net regression), because this value led to a fit that was close to the best fit and a manageable amount of LMRs. The lambda value was chosen using cross-validation on the training data as 0.3194. This identified a set of 39 LMRs together with corresponding beta values, which define the weights for these LMRs used in the chicken methylation clock. The mean squared error of 6-fold crossvalidation using the values of 0.84 for alpha and 0.3194 for lambda was 13.4831. This indicates that a new sample can be predicted with an error of about 3.7 days. In order to apply the clock to a new sample the methylation ratios of this sample at the 39 clock LMRs have to be provided and the command predict.cv of the package glmnet with the trained clock has to be performed.

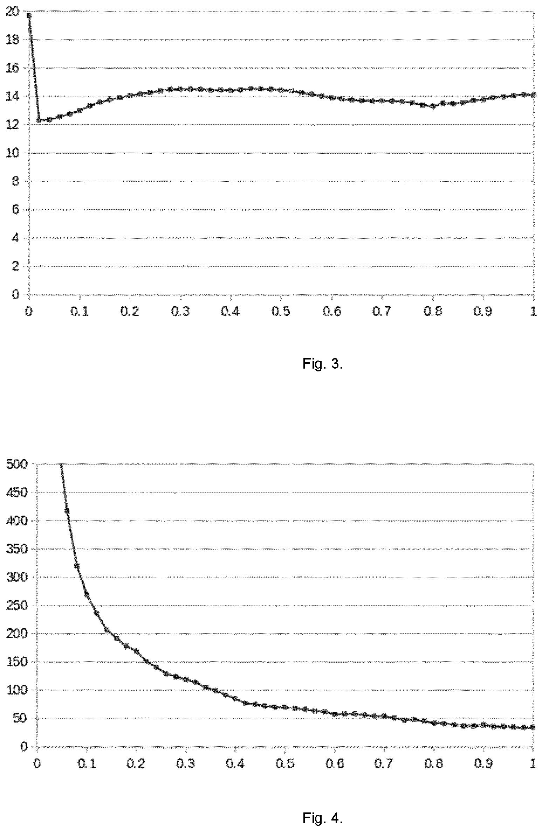

Fig. 3 shows the mean squared error of a trained clock for given alpha at value of lambda leading to the minimal error.

Fig. 4 shows the number of LMRs for given alpha at value of lambda leading to the minimal error.

**Table 2: Clock CpGs (LMR methylation, alpha = 0.84, lambda = 0.3194, #LMR's: 39).**

| ¹: Correction factors of the different tissues. The respective value has to be subtracted. | | | | | | | | |
|---|---|---|---|---|---|---|---|---|
| ID | chrom | start | end | weight | Ileum ¹ | Spleen ¹ | Breast ¹ | Jejunum ¹ |
| 1 | chr1 | 44395372 | 44398932 | -11.474 | 0.085 | 0.119 | 0.087 | 0.111 |
| 2 | chr1 | 83295508 | 83295820 | 3.676 | 0.277 | 0.463 | 0.204 | 0.305 |
| 3 | chr1 | 194750612 | 194750882 | 1.159 | 0.09 | 0.199 | 0.071 | 0.101 |
| 4 | chr2 | 8123576 | 8124320 | 3.335 | 0.179 | 0.168 | 0.113 | 0.279 |
| 5 | chr2 | 31316252 | 31316368 | 11.63 | 0.129 | 0.087 | 0.08 | 0.111 |
| 6 | chr2 | 35582600 | 35584144 | 12.066 | 0.305 | 0.357 | 0.341 | 0.317 |
| 7 | chr2 | 42878428 | 42879088 | -1.381 | 0.479 | 0.245 | 0.336 | 0.44 |
| 8 | chr2 | 63925292 | 63925632 | 7.773 | 0.086 | 0.321 | 0.117 | 0.115 |
| 9 | chr2 | 81161918 | 81161974 | 3.276 | 0.234 | 0.491 | 0.269 | 0.241 |
| 10 | chr2 | 91174539 | 91175128 | -28.595 | 0.235 | 0.262 | 0.181 | 0.238 |
| 11 | chr2 | 103673926 | 103674122 | -1.539 | 0.215 | 0.104 | 0.191 | 0.174 |
| 12 | chr3 | 77360372 | 77360404 | 1.67 | 0.152 | 0.263 | 0.1 | 0.199 |
| 13 | chr5 | 839710 | 840094 | 5.314 | 0.231 | 0.328 | 0.145 | 0.233 |
| 14 | chr5 | 1942054 | 1942842 | 1.067 | 0.325 | 0.414 | 0.23 | 0.349 |
| 15 | chr5 | 28482294 | 28482418 | 0.767 | 0.113 | 0.304 | 0.09 | 0.264 |
| 16 | chr5 | 39059306 | 39059368 | 3.441 | 0.025 | 0.068 | 0.028 | 0.058 |
| 17 | chr6 | 8416238 | 8416588 | 21.541 | 0.13 | 0.2 | 0.09 | 0.16 |
| 18 | chr7 | 5169488 | 5169670 | 2.308 | 0.232 | 0.23 | 0.244 | 0.213 |
| 19 | chr7 | 17839660 | 17839728 | -5.446 | 0.685 | 0.445 | 0.579 | 0.617 |
| 20 | chr9 | 23812488 | 23812678 | 4.227 | 0.155 | 0.382 | 0.185 | 0.151 |
| 21 | chr11 | 675297 | 675546 | -1.501 | 0.316 | 0.329 | 0.59 | 0.346 |
| 22 | chr12 | 1688020 | 1688132 | 0.37 | 0.163 | 0.359 | 0.166 | 0.213 |
| 23 | chr12 | 6875861 | 6876152 | -0.25 | 0.301 | 0.084 | 0.212 | 0.277 |
| 24 | chr12 | 10983288 | 10984278 | -0.007 | 0.258 | 0.294 | 0.303 | 0.225 |
| 25 | chr12 | 16248174 | 16248357 | -1.758 | 0.598 | 0.583 | 0.819 | 0.317 |
| 26 | chr13 | 13146982 | 13147888 | -17.978 | 0.167 | 0.113 | 0.13 | 0.179 |
| 27 | chr13 | 16017638 | 16017826 | -0.017 | 0.155 | 0.224 | 0.199 | 0.14 |
| 28 | chr13 | 16716158 | 16716440 | -0.034 | 0.153 | 0.273 | 0.147 | 0.18 |
| 29 | chr14 | 4137808 | 4137912 | -0.166 | 0.259 | 0.137 | 0.22 | 0.215 |
| 30 | chr15 | 8945392 | 8945554 | -8.922 | 0.493 | 0.464 | 0.727 | 0.324 |
| 31 | chr17 | 2483692 | 2483848 | 8.025 | 0.142 | 0.286 | 0.097 | 0.204 |
| 32 | chr17 | 3822992 | 3823290 | 2.947 | 0.207 | 0.512 | 0.206 | 0.228 |
| 33 | chr17 | 10211804 | 10212170 | -3.233 | 0.099 | 0.087 | 0.189 | 0.08 |
| 34 | chr20 | 2469403 | 2470309 | -4.959 | 0.173 | 0.273 | 0.253 | 0.262 |
| 35 | chr20 | 10704150 | 10704244 | -2.422 | 0.216 | 0.137 | 0.169 | 0.195 |
| 36 | chr20 | 11718629 | 11718916 | 3.151 | 0.149 | 0.379 | 0.23 | 0.201 |
| 37 | chr23 | 2763708 | 2763780 | 2.721 | 0.331 | 0.61 | 0.428 | 0.366 |
| 38 | chr23 | 5159782 | 5159918 | -2.9 | 0.283 | 0.171 | 0.309 | 0.228 |
| 39 | chr28 | 2874382 | 2874447 | 0.005 | 0.369 | 0.328 | 0.322 | 0.327 |
| Intercept of linear model equation found by glmnet: 17.411 | | | | | | | | |

### Example 2:

The alpha value was varied in a range between 0 and 1 and chosen as 0.9 (elastic net regression).This identified a set of 32 LMRs together with corresponding beta values, which define the weights for these LMRs used in the chicken methylation clock (Tab. 3).

**Table 3. Clock LMRs (alpha = 0.9, lambda = 0.3147).**

| ID | chrom | start | end | weight | ileum | spleen | breast | jejunum |
|---|---|---|---|---|---|---|---|---|
| 1 | chr1 | 3310966 | 3311076 | 5.106 | 0.089 | 0.117 | 0.048 | 0.108 |
| 2 | chr1 | 13486724 | 13487721 | -1.078 | 0.421 | 0.180 | 0.224 | 0.424 |
| 3 | chr1 | 77403928 | 77404268 | 5.291 | 0.106 | 0.160 | 0.040 | 0.183 |
| 4 | chr1 | 131728204 | 131729184 | -6.235 | 0.407 | 0.363 | 0.318 | 0.197 |
| 5 | chr1 | 135369614 | 135369882 | -1.194 | 0.436 | 0.184 | 0.403 | 0.419 |
| 6 | chr1 | 165806748 | 165806816 | -0.009 | 0.477 | 0.527 | 0.844 | 0.542 |
| 7 | chr2 | 31315302 | 31315823 | 0.961 | 0.148 | 0.099 | 0.104 | 0.200 |
| 8 | chr2 | 31316250 | 31316368 | 15.824 | 0.129 | 0.087 | 0.059 | 0.111 |
| 9 | chr2 | 91174537 | 91175128 | -26.554 | 0.235 | 0.262 | 0.188 | 0.238 |
| 10 | chr4 | 1489570 | 1490794 | -8.003 | 0.176 | 0.149 | 0.158 | 0.214 |
| 11 | chr4 | 8453114 | 8454528 | 3.325 | 0.159 | 0.524 | 0.316 | 0.211 |
| 12 | chr4 | 31342294 | 31342536 | 0.228 | 0.638 | 0.574 | 0.638 | 0.640 |
| 13 | chr5 | 839708 | 840094 | 2.227 | 0.231 | 0.328 | 0.153 | 0.233 |
| 14 | chr5 | 1942052 | 1942842 | 2.613 | 0.325 | 0.414 | 0.204 | 0.349 |
| 15 | chr5 | 39059304 | 39059368 | 0.307 | 0.025 | 0.068 | 0.024 | 0.058 |
| 16 | chr5 | 52951604 | 52951808 | 2.676 | 0.070 | 0.148 | 0.024 | 0.091 |
| 17 | chr6 | 8416236 | 8416588 | 12.930 | 0.130 | 0.200 | 0.099 | 0.160 |
| 18 | chr8 | 13056204 | 13056776 | 4.557 | 0.142 | 0.269 | 0.122 | 0.150 |
| 19 | chr9 | 23812486 | 23812678 | 6.756 | 0.155 | 0.382 | 0.179 | 0.151 |
| 20 | chr11 | 675295 | 675546 | -3.678 | 0.316 | 0.329 | 0.638 | 0.346 |
| 21 | chr12 | 9433040 | 9433568 | 9.905 | 0.406 | 0.351 | 0.132 | 0.409 |
| 22 | chr12 | 16248172 | 16248357 | -0.539 | 0.598 | 0.583 | 0.815 | 0.317 |
| 23 | chr13 | 13146980 | 13147888 | -10.892 | 0.167 | 0.113 | 0.135 | 0.179 |
| 24 | chr13 | 16716156 | 16716440 | -0.540 | 0.153 | 0.273 | 0.166 | 0.180 |
| 25 | chr14 | 4137806 | 4137912 | -6.589 | 0.259 | 0.137 | 0.232 | 0.215 |
| 26 | chr15 | 8945390 | 8945554 | -3.262 | 0.493 | 0.464 | 0.741 | 0.324 |
| 27 | chr18 | 2358384 | 2359684 | -2.706 | 0.448 | 0.368 | 0.364 | 0.472 |
| 28 | chr19 | 9052179 | 9052244 | -9.309 | 0.601 | 0.295 | 0.258 | 0.523 |
| 29 | chr20 | 11718627 | 11718916 | 20.167 | 0.149 | 0.379 | 0.193 | 0.201 |
| 30 | chr23 | 5568088 | 5568140 | -2.259 | 0.402 | 0.290 | 0.436 | 0.439 |
| 31 | chr25 | 1101298 | 1101396 | -0.093 | 0.493 | 0.267 | 0.204 | 0.416 |
| 32 | chr26 | 4608324 | 4608370 | 2.441 | 0.163 | 0.416 | 0.228 | 0.203 |
| Intercept of linear model equation found by glmnet: 17.345 | | | | | | | | |

Correction factors are indicated for different tissues. For correction, the corresponding value has to be subtracted.

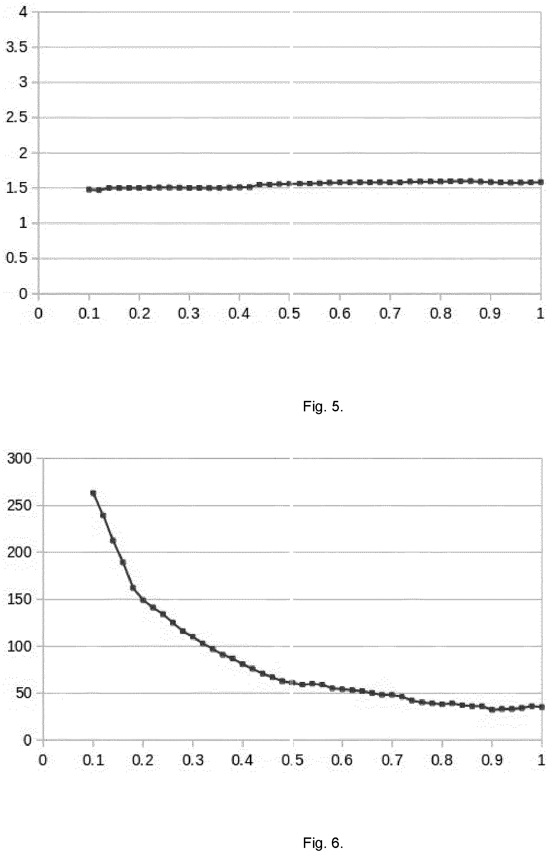

Fig. 5 shows the root mean squared error of a trained clock for given alpha at value of lambda leading to the minimal error.

Fig. 6 shows the number of LMRs for given alpha at value of lambda leading to the minimal error.

### Rationale for the normalization of the methylation data as input for the clock

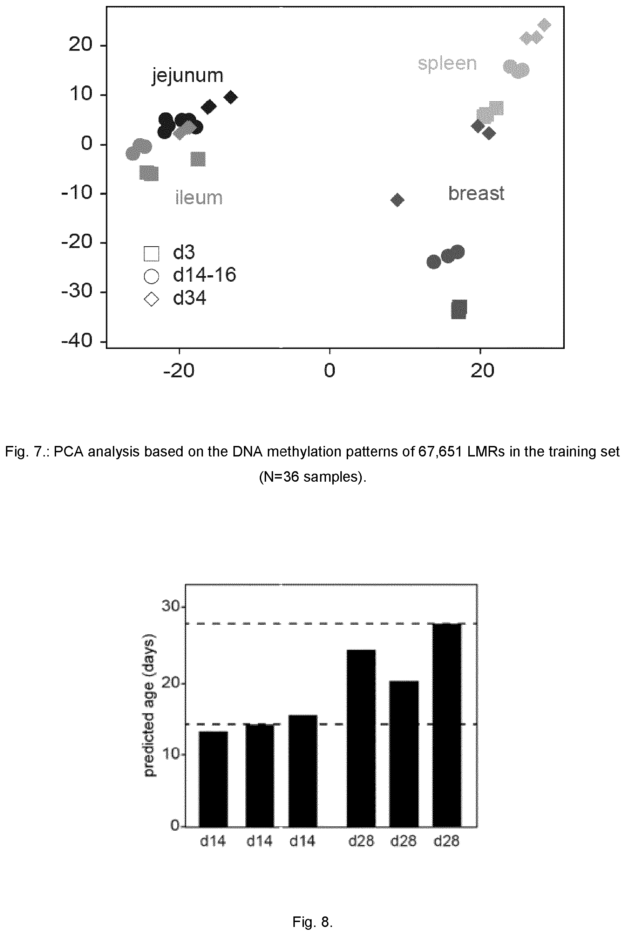

The average methylation values of these LMRs were computed and normalized by computing for every LMR the average value over all samples from the same tissue and subtracting this value from the value of this LMR (in case of the CpG clock by computing for every CpG the average value over all samples from the same tissue and subtracting this value from the value of this CpG), see above. The rationale for this approach is illustrated in Fig. 7, showing the first two principal components of a principal component analysis (PCA) of the LMR methylation data. PC2 (variance explained: 22.8%) shows a strong positive correlation with the age of the subjects (r=0.466) whereas PC1 (variance explained: 45.6%) does not show any correlation with the age of the subjects (r=-0.005). This leads to the interpretation that PC2 reflects the age of the subjects, with a higher age corresponding to a higher value of the sample on PC2. Consequently, the values of the different samples on PC2 represent an ordering of the samples with respect to age. However, even the oldest samples of breast tissue still showed a smaller value than the youngest samples of spleen tissue, although the order within the set of samples of a specific tissue is largely correct. This indicates a tissue-specific "offset" for the positioning in the age-reflecting PC2, which probably is caused by different maturation stages for different tissues at certain time points in the early life phase of chicken. As this offset is likely to affect the training of the methylation clock algorithm, the corresponding correction was introduced.

### Age prediction in breast tissue from a completely independent validation dataset:

In order to validate the LMR clock, whole-genome bisulfite sequencing of 6 samples (breast) in two age groups (14 and 28 days) from a completely independent animal trial was performed. Age prediction showed a root mean square error of 2.7 days and 3.8 days, respectively, which is consistent with the prediction error obtained after cross-validation. Results are visualized in Fig. 8.

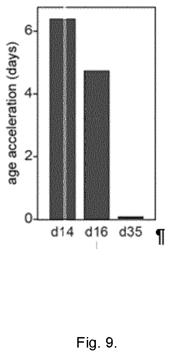

Analysis of jejunum samples showed a pronounced and highly consistent age acceleration, in particular at days 14 and 16 (Fig. 9). A control group was injected with the non-inflammatory agent GpC and did not respond at all.

## Claims

1. A computer-implemented method of establishing an epigenetic clock for an avian species, the method comprising
(a.) identifying and determining the methylation levels of specific CpG sites within the genomic DNA obtained from a plurality of different biological sample materials deriving from the avian species and representing specific time points within the chronological lifespan of this avian species,
(b.) excluding all CpG sites associated with single nucleotide polymorphisms from the CpG sites identified in step (a.),
(c.) excluding all CpG sites located on the sex chromosomes from the CpG sites obtained in step (b.),
(d.) performing a tissue-specific normalization step for the CpG sites obtained in step (c.), and
(e.) correlating the CpG methylation levels of the CpG sites obtained in step (d.) with chronological age using a penalized regression model,
wherein the specific CpG sites within the genomic DNA of the avian species are distributed within low methylated regions (LMRs) in the genome of the avian species..

2. The method according to claim 1, wherein the plurality of different biological sample materials deriving from the avian species and representing specific time points within the chronological lifespan of this avian species includes material selected from the group consisting of body fluids, excremental material, tissue material and feather material.

3. The method according to claim 1, wherein plurality of different biological sample materials deriving from the avian species and representing specific time points within the chronological lifespan of this avian species includes at least four different tissues.

4. The method according to any one of the preceding claims, wherein the plurality of different biological sample materials deriving from the avian species and representing specific time points within the chronological lifespan of this avian species comprises or consists of tissue material selected from muscle tissue, gut tissue, organ tissue and skin tissue.

5. The method according to any one of the preceding claims, wherein the plurality of different biological sample materials deriving from the avian species and representing specific time points within the chronological lifespan of this avian species includes breast tissue, spleen tissue, ileum tissue and jejunum tissue.

6. The method according to any one of the preceding claims, wherein the plurality of different biological sample materials deriving from the avian species and representing specific time points within the chronological lifespan of this avian species is selected to represent ages ranging between 3 days and 63 days.

7. The method according to any one of the preceding claims, wherein step (a) involves a whole-genome bisulfite sequencing process.

8. The method according to any one of the preceding claims, wherein the specific CpG sites within the genomic DNA of the avian species are distributed over the whole genome of the avian species and were restricted to a strand-specific coverage of at least 10.

9. The method according to claim 8, wherein the tissue-specific normalization step is performed by computing for every CpG the average value over all samples from the same tissue and subtracting this value from the value of this CpG.

10. The method according to claim 1, wherein the specific CpG sites within the genomic DNA of the avian species were restricted to a strand-specific coverage of at least greater than 5.

11. The method according to claim 1, wherein the tissue-specific normalization step is performed by computing for every LMR the average value over all samples from the same tissue and subtracting this value from the value of this LMR.

12. A computer program loaded into a memory of a computer, implementing the method of any one of claims 1 to 9.

13. A tangible, computer-readable medium comprising a computer-readable code that, when executed by a computer, causes the computer to perform operations comprising:
(a.) receiving information corresponding to the methylation levels of specific CpG sites within the genomic DNA of the avian species obtained from a plurality of different biological sample materials representing specific time points within the chronological lifespan of the avian species,
(b.) receiving information corresponding to all CpG sites associated with single nucleotide polymorphisms, and excluding same from the CpG sites of step (a.),
(c.) receiving information corresponding to all CpG sites from the sex chromosomes and excluding same from the CpG sites of step (b.),
(d.) performing a tissue-specific normalization step for the CpG sites of step (c.), and
(e.) correlating the CpG methylation levels of the CpG sites of step (d.) with chronological age using a penalized regression model,
wherein the specific CpG sites within the genomic DNA of the avian species are distributed within low methylated regions (LMRs) in the genome of the avian species.

## Patentansprüche

1. Computergestütztes Verfahren zum Einrichten einer epigenetischen Uhr für eine Vogelspezies, wobei das Verfahren Folgendes umfasst:
(a.) Identifizieren und Bestimmen der Methylierungsniveaus spezifischer CpG-Stellen innerhalb der genomischen DNA, die aus mehreren unterschiedlichen biologischen Probenmaterialien, die von der Vogelspezies stammen und spezifische Zeitpunkte innerhalb der chronologischen Lebensspanne dieser Vogelspezies darstellen, erhalten wurde,
(b.) Ausschließen aller mit Einzelnukleotidpolymorphismen assoziierten CpG-Stellen von den in Schritt (a.) identifizierten CpG-Stellen,
(c.) Ausschließen aller sich auf den Geschlechtschromosomen befindenden CpG-Stellen von den in Schritt (b.) erhaltenen CpG-Stellen,
(d.) Durchführen eines gewebespezifischen Normalisierungsschritts für die in Schritt (c.) erhaltenen CpG-Stellen und
(e.) Korrelieren der CpG-Methylierungsniveaus der in Schritt (d.) erhaltenen CpG-Stellen mit dem chronologischen Alter unter Verwendung eines penalisierten Regressionsmodells,
wobei die spezifischen CpG-Stellen innerhalb der genomischen DNA der Vogelspezies innerhalb von niedrig methylierten Regionen (Low Methylated Regions, LMR) im Genom der Vogelspezies verteilt sind.

2. Verfahren nach Anspruch 1, wobei die mehreren unterschiedlichen biologischen Probenmaterialien, die von der Vogelspezies stammen und spezifische Zeitpunkte innerhalb der chronologischen Lebensspanne dieser Vogelspezies darstellen, Material umfassen, das aus der Gruppe bestehend aus Körperflüssigkeiten, Ausscheidungsmaterial, Gewebematerial und Federmaterial ausgewählt ist.

3. Verfahren nach Anspruch 1, wobei mehrere unterschiedliche biologische Probenmaterialien, die von der Vogelspezies stammen und spezifische Zeitpunkte innerhalb der chronologischen Lebensspanne dieser Vogelspezies darstellen, mindestens vier verschiedene Gewebe umfassen.

4. Verfahren nach einem der vorhergehenden Ansprüche, wobei die mehreren unterschiedlichen biologischen Probenmaterialien, die von der Vogelspezies stammen und spezifische Zeitpunkte innerhalb der chronologischen Lebensspanne dieser Vogelspezies darstellen, Gewebematerial, das aus Muskelgewebe, Darmgewebe, Organgewebe und Hautgewebe ausgewählt ist, umfassen bzw. daraus bestehen.

5. Verfahren nach einem der vorhergehenden Ansprüche, wobei die mehreren unterschiedlichen biologischen Probenmaterialien, die von der Vogelspezies stammen und spezifische Zeitpunkte innerhalb der chronologischen Lebensspanne dieser Vogelspezies darstellen, Brustgewebe, Milzgewebe, Ileumgewebe und Jejunumgewebe umfassen.

6. Verfahren nach einem der vorhergehenden Ansprüche, wobei die mehreren unterschiedlichen biologischen Probenmaterialien, die von der Vogelspezies stammen und spezifische Zeitpunkte innerhalb der chronologischen Lebensspanne dieser Vogelspezies darstellen, so ausgewählt sind, dass sie Alter im Bereich zwischen 3 Tagen und 63 Tagen repräsentieren.

7. Verfahren nach einem der vorhergehenden Ansprüche, wobei Schritt (a) ein Gesamtgenom-Hydrogensulfit-Sequenzierverfahren beinhaltet.

8. Verfahren nach einem der vorhergehenden Ansprüche, wobei die spezifischen CpG-Stellen innerhalb der genomischen DNA der Vogelspezies über das Gesamtgenom der Vogelspezies verteilt sind und auf eine strangspezifische Abdeckung von wenigstens 10 beschränkt waren.

9. Verfahren nach Anspruch 8, wobei der gewebespezifische Normierungsschritt durchgeführt wird, indem für jede CpG der Durchschnittswert über alle Proben aus demselben Gewebe berechnet und dieser Wert vom Wert dieser CpG subtrahiert wird.

10. Verfahren nach Anspruch 1, wobei die spezifischen CpG-Stellen innerhalb der genomischen DNA der Vogelspezies auf eine strangspezifische Abdeckung von wenigstens größer 5 beschränkt waren.

11. Verfahren nach Anspruch 1, wobei der gewebespezifische Normierungsschritt durchgeführt wird, indem für jede LMR der Durchschnittswert über alle Proben aus demselben Gewebe berechnet und dieser Wert vom Wert dieser LMR subtrahiert wird.

12. Computerprogramm, geladen in einen Speicher eines Computers, das das Verfahren nach einem der Ansprüche 1 bis 9 ausführt.

13. Greifbares, computerlesbares Medium, umfassend einen computerlesbaren Code, der bei seiner Ausführung durch einen Computer bewirkt, dass der Computer Operationen durchführt, die Folgendes umfassen:
(a.) Empfangen von Informationen, die den Methylierungsniveaus spezifischer CpG-Stellen innerhalb der genomischen DNA der Vogelspezies entsprechen, die aus mehreren unterschiedlichen biologischen Probenmaterialien, die spezifische Zeitpunkte innerhalb der chronologischen Lebensspanne der Vogelspezies darstellen, erhalten wurde,
(b.) Empfangen von Informationen, die allen CpG-Stellen entsprechen, die mit Einzelnukleotidpolymorphismen assoziiert sind, und Ausschließen derselben von den CpG-Stellen aus Schritt (a.),
(c.) Empfangen von Informationen, die allen CpG-Stellen aus den Geschlechtschromosomen entsprechen, und Ausschließen derselben von den CpG-Stellen aus Schritt (b.),
(d.) Durchführen eines gewebespezifischen Normierungsschritts für die CpG-Stellen aus Schritt (c.) und
(e.) Korrelieren der CpG-Methylierungsniveaus der CpG-Stellen aus Schritt (d.) mit dem chronologischen Alter unter Verwendung eines penalisierten Regressionsmodells, wobei die spezifischen CpG-Stellen innerhalb der genomischen DNA der Vogelspezies innerhalb von niedrig methylierten Regionen (LMR) im Genom der Vogelspezies verteilt sind.

## Revendications

1. Procédé mis en œuvre par ordinateur d'établissement d'une horloge épigénétique pour une espèce aviaire, le procédé comprenant
(a.) l'identification et la détermination des taux de méthylation de sites CpG spécifiques dans l'ADN génomique obtenu à partir d'une pluralité de matériaux d'échantillons biologiques différents provenant de l'espèce aviaire et représentant des moments spécifiques de la vie chronologique de cette espèce aviaire,
(b.) l'exclusion de tous les sites CpG associés à des polymorphismes mononucléotidiques des sites CpG identifiés à l'étape (a.),
(c.) l'exclusion de tous les sites CpG situés sur les chromosomes sexuels des sites CpG obtenus à l'étape (b.),
(d.) la réalisation d'une étape de normalisation spécifique à un tissu pour les sites CpG obtenus à l'étape (c.), et
(e.) la corrélation des taux de méthylation des sites CpG obtenus à l'étape (d.) avec l'âge chronologique à l'aide d'un modèle de régression pénalisée,
dans lequel les sites CpG spécifiques dans l'ADN génomique de l'espèce aviaire sont répartis dans des régions faiblement méthylées (LMR) du génome de l'espèce aviaire.

2. Procédé selon la revendication 1, dans lequel la pluralité d'échantillons biologiques différents provenant de l'espèce aviaire et représentant des moments spécifiques de la vie chronologique de cette espèce aviaire comprend des matériaux choisis parmi les fluides corporels, les excréments, les tissus et les plumes.

3. Procédé selon la revendication 1, dans lequel la pluralité de matériaux d'échantillons biologiques différents provenant de l'espèce aviaire et représentant des moments spécifiques de la vie chronologique de cette espèce aviaire comprend au moins quatre tissus différents.

4. Procédé selon l'une quelconque des revendications précédentes, dans lequel la pluralité d'échantillons biologiques différents provenant de l'espèce aviaire et représentant des moments spécifiques de la vie chronologique de cette espèce aviaire comprend ou est constituée de tissus choisis parmi les tissus musculaire, intestinal, organique et cutané.

5. Procédé selon l'une quelconque des revendications précédentes, dans lequel la pluralité d'échantillons biologiques différents provenant de l'espèce aviaire et représentant des moments spécifiques de la vie chronologique de cette espèce aviaire comprend les tissus mammaire, splénique, iléal et jéjunal.

6. Procédé selon l'une quelconque des revendications précédentes, dans lequel la pluralité d'échantillons biologiques différents provenant de l'espèce aviaire et représentant des moments spécifiques de la vie chronologique de cette espèce aviaire est choisie de façon à représenter les âges compris entre 3 jours et 63 jours.

7. Procédé selon l'une quelconque des revendications précédentes, dans lequel l'étape (a) implique un processus de séquençage au bisulfite du génome entier.

8. Procédé selon l'une quelconque des revendications précédentes, dans lequel les sites CpG spécifiques dans l'ADN génomique de l'espèce aviaire sont répartis sur l'ensemble du génome de l'espèce aviaire et sont limités à une couverture spécifique à un brin supérieure ou égale à 10.

9. Procédé selon la revendication 8, dans lequel l'étape de normalisation spécifique à un tissu est réalisée par calcul, pour chaque CpG, de la valeur moyenne pour tous les échantillons du même tissu et soustraction de cette valeur de la valeur de ce CpG.

10. Procédé selon la revendication 1, dans lequel les sites CpG spécifiques dans l'ADN génomique de l'espèce aviaire sont limités à une couverture spécifique à un brin supérieure ou égale à 5.

11. Procédé selon la revendication 1, dans lequel l'étape de normalisation spécifique à un tissu est réalisée par calcul, pour chaque LMR, de la valeur moyenne pour tous les échantillons du même tissu et soustraction de cette valeur de la valeur de cette LMR.

12. Programme informatique chargé dans la mémoire d'un ordinateur, mettant en œuvre le procédé selon l'une quelconque des revendications 1 à 9.

13. Support tangible, lisible par ordinateur, comprenant un code lisible par ordinateur qui, lorsqu'il est exécuté par un ordinateur, amène l'ordinateur à effectuer des opérations comprenant :
(a.) la réception d'informations correspondant aux taux de méthylation de sites CpG spécifiques dans l'ADN génomique de l'espèce aviaire, obtenues à partir d'une pluralité d'échantillons biologiques différents représentant des moments spécifiques de la vie chronologique de l'espèce aviaire,
(b.) la réception d'informations correspondant à tous les sites CpG associés à des polymorphismes mononucléotidiques et leur exclusion des sites CpG de l'étape (a.),
(c.) la réception d'informations correspondant à tous les sites CpG situés sur les chromosomes sexuels et leur exclusion des sites CpG de l'étape (b.),
(d.) la réalisation d'une étape de normalisation spécifique à un tissu pour les sites CpG de l'étape (c.), et
(e.) la corrélation des taux de méthylation des sites CpG de l'étape (d.) avec l'âge chronologique à l'aide d'un modèle de régression pénalisée,
les sites CpG spécifiques dans l'ADN génomique de l'espèce aviaire étant répartis dans des régions faiblement méthylées (LMR) du génome de l'espèce aviaire.
